# FASCICULE DE BREVET EUROPEEN

(11) **EP 0 447 318 B1**
(45) Date de publication et mention de la délivrance du brevet: **31.05.1995**
(21) Numéro de dépôt: 91400684.6
(22) Date de dépôt: 13.03.1991
(51) Int. Cl.: A61K 9/51, A61K 7/00

(54) **Composition pour le traitement cosmétique et/ou pharmaceutique des couches supérieures de l'épiderme par application topique sur la peau et procédé de préparation correspondant**
Zusammensetzung zur kosmetischen und/oder pharmazeutischen Behandlung der obersten Epidermisschicht durch topische Anwendung auf der Haut und das entsprechende Herstellungsverfahren
Composition for the cosmetic and/or pharmaceutical treatment of the surface epidermal layers by topical application on the skin and the relative production process

(30) Priorité: 16.03.1990 FR 9003418
(43) Date de publication de la demande: 18.09.1991
(73) Titulaire: L'OREAL, 75008 Paris (FR)
(72) Inventeur: Handjani, Rose-Marie, F-75014 Paris (FR); Ribier, Alain, F-75014 Paris (FR)
(74) Mandataire: Peuscet, Jacques

(56) Documents cités:
- EP-A- 0 274 961
- EP-A- 0 330 180
- FR-A- 2 515 960
- US-A- 3 965 033

## Description

La présente invention concerne une composition pour le traitement cosmétique et/ou pharmaceutique des couches supérieures de l'épiderme, par application topique de ladite composition sur la peau, et un procédé de préparation pour l'obtenir.

Il est bien connu, en cosmétique et/ou en pharmacie, d'appliquer sur la peau des huiles actives et/ou contenant un actif. Ces huiles sont utilisées telles quelles ou, le plus souvent, sous forme d'émulsion eau dans l'huile ou huile dans l'eau. On sait que ces huiles ont une action sur la surface de la peau, mais également dans les couches supérieures de l'épiderme, car elles traversent le stratum corneum, l'action cosmétique et/ou pharmaceutique des huiles étant généralement d'autant plus efficace qu'une proportion plus grande d'huile pénètre dans les couches supérieures de l'épiderme.

Par ailleurs, on connaît les nanoparticules: on désigne sous ce nom des particules colloïdales, dont la taille est de l'ordre de 10 à 1000 nm et qui sont constituées de matériaux polymériques, dans lesquelles un principe actif est piégé, encapsulé et/ou adsorbé (voir J. KREUTER, J. MICROENCAPSULATION 1988, Vol. 5, pages 115-127). Sous le terme de nanoparticules, on peut désigner des nanosphères et des nanocapsules : une nanosphère constitue une matrice polymérique solide poreuse sur laquelle l'actif est adsorbé ; une nanocapsule est une membrane polymérique qui entoure un coeur constitué par le principe actif. Pour la suite de la description et dans les revendications, on limitera strictement la portée du terme "nanoparticules" aux nanocapsules ci-dessus définies. Parmi les polymères utilisables pour la fabrication de nanoparticules, on choisit, de préférence, les matériaux biodégradables afin de permettre l'utilisation thérapeutique desdites nanoparticules. On sait que les cyanoacrylates, et en particulier les polyalkyl cyanoacrylates, permettent d'obtenir des nanoparticules biodégradables ; la préparation de nanoparticules à partir de cyanoacrylates est décrite dans EP-B 0 007 895 et EP-B 0 064 967.

L'usage des nanoparticules biodégradables encapsulant des composés biologiquement actifs a été proposé, dans de nombreuses applications thérapeutiques, pour de nombreux principes actifs tels que des substances antimitotiques ou antinéoplasiques, des antibiotiques, des substances hormonales, l'insuline, l'héparine ou des produits biologiques comme des protéines, des antigènes ou les constituants de virus, de bactéries ou de cellules. On a donc déjà proposé d'administrer des nanoparticules encapsulant des principes actifs par voie orale, sous-cutanée, intradermique, intramusculaire, intraveineuse, et par application oculaire (voir EP-B 0 007 895, EP-B-0 064 967, FR-B-2 604 903, DE-A-3 722 837, DE-A-3 341 001, FR-B-2 304 326).

Dans FR-A-2 515 960, on a décrit des nanoparticules de cyanoacrylate encapsulant une huile ou une substance active dispersée dans une huile et il est précisé que ces nanoparticules peuvent être administrées par voie orale, sous-cutanée, intradermique, intramusculaire ou intraveineuse. En outre, dans ce document, on a décrit l'utilisation de nanoparticules pour encapsuler des parfums, les parfums encapsulés ayant l'avantage de faire persister l'odeur parfumée plus longtemps après l'application que dans le cas où l'on applique le parfum sur la peau sans encapsulation ; l'encapsulation a donc une action retard sur le parfum. De plus, dans ce cas, l'action recherchée du parfum se fait à la surface de la peau et la persistance de l'odeur est tout à fait indépendante du sort de la fraction des nanoparticules qui, éventuellement, pourraient traverser le stratum corneum. Cette application topique ne fournit donc aucune information quant à l'éventuelle capacité des nanoparticules à traverser le stratum corneum et à se dégrader dans les couches supérieures de l'épiderme ; elle laisse, au contraire, présager que les nanoparticules resteraient en majeure partie à la surface de la peau, puisqu'elles permettent le dégagement du parfum.

Selon la présente demande, on a trouvé que, par application topique cutanée d'une composition comprenant des nanoparticules biodégradables encapsulant des huiles actives et/ou contenant un actif, on obtenait une action cosmétique et/ou pharmaceutique particulièrement efficace.

L'amélioration de l'efficacité cosmétique et/ou pharmaceutique des huiles grâce à l'encapsulation dans des nanoparticules n'était aucunement prévisible ; il n'était pas évident, en effet, d'une part, que les nanoparticules traverseraient plus facilement le stratum corneum que l'huile non encapsulée, qu'elle soit sous forme d'émulsion eau dans l'huile ou huile dans l'eau, et, d'autre part, que ces nanoparticules seraient biodégradées au niveau des couches supérieures de l'épiderme. On savait, certes, que les formes proposées d'administration dans le corps humain, en particulier par injection, conduisent à la biodégradation des nanoparticules dans les différentes zones de tissus où elles sont introduites. Mais, il est bien connu que les différents tissus ont des constitutions différentes et contiennent des enzymes différents ; en particulier, il est bien connu que le tissu conjonctif du muscle, le derme et les couches profondes de l'épiderme, où les nanoparticules avaient antérieurement été introduites par injection, ont une constitution bio-chimique très différente de celle des couches supérieures de l'épiderme (voir, par exemple, British Journal of Dermatology (1976) 94,443).

Rien ne permettait donc à l'homme de métier de prévoir que les nanoparticules étaient, d'une part, susceptibles de traverser le stratum corneum en quantité significative et, d'autre part, susceptibles de libérer très rapidement l'actif dans les couches supérieures de l'épiderme.

Selon la présente invention, on a trouvé, en outre, que, l'encapsulation d'une huile active (ou contenant un actif) dans des nanoparticules produit, de façon surprenante, une action immédiate de la composition. L'effet retard annoncé pour les compositions parfumantes appliquées par voie topique dans le document FR-A-2 515 960 précité, conduisait l'homme de métier à prévoir qu'une application par voie topique, à supposer qu'elle puisse avoir un effet, ne pouvait avoir qu'une action retardée. L'invention propose donc une composition, dont les propriétés sont inattendues. Par ailleurs, ces propriétés son particulièrement bien adaptées à la voie topique, comme l'a montré une étude comparative in vitro d'absorption percutanée.

La présente invention a, en conséquence, pour premier objet l'utilisation de nanoparticules polymères biodégradables encapsulant au moins un actif sous forme d'huile et/ou au moins un actif contenu dans une huile-support non active ou une huile active, l'actif étant choisi parmi ceux ayant une action pharmaceutique contre une affection se manifestant dans les couches supérieures de l'épiderme, pour l'obtention d'un médicament pour le traitement des couches supérieures de l'épiderme par application topique sur la peau.

La présente invention a pour second objet un procédé de traitement cosmétique des couches supérieures de l'épiderme par application topique sur la peau, caractérisé par le fait que l'on applique sur la peau une composition contenant, dans un véhicule approprié, des nanoparticules polymères biodégradables encapsulant au moins un actif sous forme d'huile et/ou au moins un actif contenu dans une huile-support non active ou une huile active, l'actif étant choisi parmi ceux ayant une action cosmétique sur les couches supérieures de l'épiderme.

Les nanoparticules utilisées ont, de préférence, des dimensions comprises entre 10 et 1000nm et, plus particulièrement, entre 50 et 500nm.

Le poids des nanoparticules chargées d'au moins un actif constitue avantageusement de 0,1% à 20% du poids total de la composition et, de préférence, de 0,5 à 5% en poids.

Les polymères constituant les nanoparticules biodégradables peuvent être des polymères de cyanoacrylate d'alkyle en C₂-C₁₂ et, en particulier, en C₂-C₆; le radical alkyle est, de préférence, choisi dans le groupe formé par les radicaux éthyle, n-butyle, hexyle, isobutyle et isohexyle. Les polymères biodégradables peuvent aussi être pris dans le groupe formé par les poly-L-lactides, les poly-DL-lactides, les polyglycolides, les polycaprolactones, les polymères de l'acide 3-hydroxy butyrique et les copolymères correspondants, tels que les copoly(DL-lactides et glycolides), copoly(glycolides et caprolactones) et similaires.

L'utilisation des nanoparticules obtenues à partir de poly-L-lactides, de poly-DL-lactides et de copoly(DL-lactides et glycolides) est particulièrement avantageuse car les produits de biodégradation enzymatique ou chimique de ces nanoparticules peuvent avoir eux-mêmes des effets cosmétiques: ainsi l'acide lactique présente des propriétés humectantes et plastifiantes; l'acide glycolique présente des propriétés dépigmentantes et/ou biostimulantes.

Les actifs sous forme d'huile (ou huiles actives) sont, de préférence, choisis dans le groupe formé par l'α-tocophérol, l'acétate d'α-tocophérol, les triglycérides riches en acide linoléique et/ou linolénique, le tétraéthyl-2-hexanoate de pentaérythritol, le clofibrate, le linoléate de tocophérol, l'huile de poisson, l'huile de noisette, le bisabolol, le farnesol, l'acétate de farnesyl, le linoléate d'éthyle et le paramethoxycinnamate d'éthyle hexyle.

Les huiles-supports non actives sont, de préférence, choisies dans le groupe formé par les triglycérides simples ou modifiés, en particulier par oxyéthylénation, les huiles silicone volatiles et leurs mélanges.

Pour obtenir les nanoparticules chargées utilisées dans la composition selon l'invention, on peut, soit prendre, une huile active, soit introduire, dans une huile active ou dans une huile-support non active par elle-même, tout actif susceptible d'avoir une activité cosmétique ou thérapeutique. Ces actifs peuvent être, entre autres, des émollients, des humectants, des agents antiradicalaires, des antiinflammatoires, des vitamines, des dépigmentants, des antiacnéiques, des antiséborrhéiques, des kératolytiques, des amincissants, des agents de coloration de la peau, des filtres solaires, et, notamment, l'acide linoléique, le rétinol, l'acide rétinoïque, les alkylesters d'acide ascorbique, les acides gras polyinsaturés, les esters nicotiniques, le nicotinate de tocophérol, les insaponifiables de riz, de soja, de karité, les céramides, les hydroxyacides tels que l'acide glycolique, les dérivés de sélénium, les antioxydants, le β-carotène, le γ-orizanol et le glycérate de stéaryle.

L'actif est, de préférence, un actif oléophile sous forme de solution dans l'huile. Mais il peut être également sous forme de dispersion, de suspension ou d'émulsion.

Dans les nanoparticules, le rapport en poids entre le polymère biodégradable des nanoparticules et la phase huileuse active est, de préférence, compris entre 0,05 et 0,5 et, notamment, voisin de 0,2.

Les compositions selon l'invention peuvent se présenter sous forme de sérum, de lotion, de gel aqueux, hydroalcoolique ou huileux, ou d'émulsion eau dans l'huile ou huile dans l'eau ou encore de dispersions aqueuses de vésicules, dont les lipides constitutifs sont des lipides ioniques ou des lipides non-ioniques ou un mélange de lipides ioniques et non-ioniques, avec ou sans phase huileuse. Leur utilisation pour constituer des sérums est particulièrement avantageuse : en effet, ce type de produit nécessite l'introduction d'une quantité importante d'émulsionnant dans le cas ou l'on souhaite y introduire des actifs huileux non encapsulés et il est bien connu que les émulsionnants ont pour effet d'irriter la peau et ne sont pas compatibles avec tous les actifs.

Les compositions peuvent contenir, en plus des nanoparticules, des adjuvants cosmétiquement et/ou pharmaceutiquement acceptables connus, tels que des corps gras, de la vaseline, des conservateurs, des agents épaississants, des colorants, des parfums.

Lorsque l'on utilise un polymère de cyanoacrylate d'alkyle (C₂ - C₁₂), pour obtenir les nanoparticules de la composition, on peut effectuer une polymérisation interfaciale d'une micro-émulsion d'huile dans un milieu hydroalcoolique, tel que décrit par exemple dans FR-A-2 515 960, en injectant, dans une phase aqueuse contenant ou non un agent tensio-actif, un mélange constitué par l'(les) huile(s) à encapsuler, au moins un cyanoacrylate d'alkyle (C₂ - C₁₂) et au moins un solvant pouvant contenir un agent tensio-actif, puis en évaporant le solvant et, éventuellement en concentrant la dispersion aqueuse de nanoparticules obtenue. Le solvant utilisé est, le plus souvent, un alcool inférieur en C₂ - C₄, en particulier l'éthanol, le propanol, l'isopropanol ou un mélange de ces alcools ou encore l'acétone ; il peut éventuellement contenir un agent tensio-actif.

On peut aussi utiliser le procédé de fabrication de nanoparticules décrit dans la demande de brevet européen n° 0 274 961. Dans ce cas, les nanoparticules sont obtenues par précipitation du polymère autour d'une dispersion de gouttelettes huileuses en injectant dans une phase aqueuse contenant ou non un agent tensioactif, un mélange constitué par l' (les) huile(s) à encapsuler, au moins un polymère et au moins un solvant contenant ou non un agent tensio-actif, puis en évaporant le solvant.

On pourrait aussi utiliser d'autres procédés.

L'agent tensio-actif éventuellement utilisé dans le procédé de préparation peut être constitué d'au moins un tensio-actif non-ionique, plus particulièrement choisi parmi les condensats de glycérol, d'oxyde d'éthylène et d'oxyde de propylène ou d'au moins un tensio-actif ionique pouvant notamment être pris dans le groupe des phospholipides, tel que la lécithine ou encore d'un mélange d'au moins un tensio-actif de chacune de ces deux catégories. Cet agent tensio-actif favorise la formation de la microémulsion d'huile et empêche la coalescence des nanoparticules au sein du mélange réactionnel. Le rapport pondéral entre l'agent tensio-actif utilisé, d'une part, et les matériaux constitutifs des nanoparticules chargées d'actif(s), d'autre part, est avantageusement compris entre 0,01 et 0,5 et, de préférence, voisin de 0,2.

Lorsqu'un agent tensio-actif utilisé au cours du procédé de fabrication des nanoparticules est par lui-même susceptible de former des vésicules constituées de feuillets lipidiques encapsulant un volume fermé, ledit agent se comporte, pour l'essentiel différemment suivant qu'il est introduit dans la phase aqueuse ou dans la phase solvant. Si l'agent tensio-actif est dans la phase aqueuse, il a tendance, au moins partiellement, à former des vésicules. Si, au contraire, l'agent tensio-actif est dans la phase solvant, il a tendance, au moins partiellement, à former autour de la membrane polymérique de chaque nanoparticules un ou plusieurs feuillet(s) lipidique(s), constitué(s) chacun d'une bi-couche moléculaire.

Dans le cas où l'huile à encapsuler est une huile autoémulsionnable, par exemple choisie parmi les triglycérides oxyéthylénés, il n'est pas nécessaire d'utiliser un agent tensio-actif.

La dispersion aqueuse de nanoparticules obtenue peut être utilisée telle quelle. Elle peut être également lyophilisée, notamment en présence d'additifs anti-mottage tels que les silices, les sucres, les sels, les protéines, les peptides et les acides aminés. Les lyophilisats ont l'avantage de permettre de préparer des compositions cosmétiques anhydres. Si les nanoparticules sont enrobées d'au moins un feuillet lipidique constitué d'au moins un agent tensio-actif susceptible de former des vésicules, les compositions selon l'invention peuvent présenter des caractéristiques cosmétiques particulièrement avantageuses. Ces nanoparticules enrobées peuvent ne constituer qu'une partie des nanoparticules de la composition.

Les exemples, donnés ci-après, à titre purement illustratif permettront de mieux comprendre l'invention.

### EXEMPLE 1 :

Dans cet exemple, on a étudié, à titre comparatif, une dispersion aqueuse de nanoparticules de polymère de cyanoacrylate de butyle contenant de l'α-tocophérol, préparée selon le mode opératoire de l'exemple 3, dans une phase aqueuse contenant un agent tensio-actif et de l'éthanol, et deux émulsions d'α-tocophérol dans l'eau obtenues à l'aide de deux agents émulsionnants différents. On a ensuite mesuré l'irritation de la peau (IRP) et l'irritation oculaire (IO).

Les mesures d'IRP ont été effectuées selon le protocole décrit dans l'arrêté ministériel du 1er Février 1982 publié au Journal Officiel de la République Française (J.O.R.F.) en date du 21 Février 1982.

Les mesures d'IO ont été effectuées selon le protocole décrit dans l'arrêté ministériel du 21 Septembre 1984 publié au J.O.R.F. des 24 Octobre 1984 et 9 Février 1985.

Les compositions et résultats sont donnés dans le tableau I.

**TABLEAU I**

| Composition en % | Nanoparticules selon l'invention | Emulsion N° 1 non conforme à l'invention | Emulsion N° 2 non conforme à l'invention |
|---|---|---|---|
| - α-tocophérol | 1,0 | 1,0 | 1,0 |
| - Butylcyano acrylate | 0,2 | - | - |
| - Solubilisant S 12 | - | 2,0 | - |
| - Solulan 16 | - | - | 2,0 |
| - Pluronic F 68 | 0,2 | - | - |
| - Kathon CG | 0,1 | 0,1 | 0,1 |
| - Eau | qs 100 | qs 100 | qs 100 |

| Nature de la mesure | Résultats | | |
|---|---|---|---|
| IRP | 0,08 | 0,29 | 2,0 |
| IO | 2,6 à 1 H | 11,83 à 1 H | 5,67 à 1 H |
| " | 1,0 à 1 J | 1,67 à 1 J | 2,33 à 1 J |
| " | 0,0 à 2 J | 0,67 à 2 J | 1,33 à 2 J |
| " | | 0,0 à 3 J | 0,0 à 3 J |

Dans ce tableau I :
- H signifie heure et J signifie jour.
- "Solubilisant S12"® est un agent émulsionnant constitué par un nonylphénol oxyéthyléné à 12 moles d'oxyde d'éthylène, vendu par la société "GIVAUDAN".
- "Solulan 16"® est un agent émulsionnant constitué par un mélange (alcools de lanoline - alcools gras oxyéthylénés à 16 moles d'oxyde d'éthylène) vendu par la société "AMERCHOL".
- "Pluronic F68"® est un agent tensio-actif constitué par un condensat d'oxyde d'éthylène et d'oxyde de propylène, vendu par la société "BASF".
- "KATHON CG"® est un conservateur constitué par un mélange : de chloro-5 méthyl-2 isothiazoline-4-one -3, de méthyl-2 isothiazoline-4-one-3 et de chlorure et nitrate de magnésium, vendu par la société "ROHM et HAAS".

Selon ce tableau, on voit que, pour obtenir les émulsions 1 et 2, 2% d'agent émulsionnant ont été nécessaires pour microdisperser 1% d'α-tocophérol. D'autre part, on voit que la composition à base de nanoparticules se révèle nettement moins irritante pour la peau que les deux émulsions et que l'irritation oculaire (IO) est également réduite aussi bien après une heure qu'après deux jours.

### EXEMPLE 2 :

Dans cet autre exemple comparatif, on a préparé, selon le mode opératoire de l'exemple 4, une dispersion aqueuse de nanoparticules selon l'invention ayant la composition pondérale suivante :

| | |
|---|---|
| - α-tocophérol | 5 % |
| - Cyanoacrylate de butyle | 1 % |
| - Tensio-actif vendu sous la dénomination commerciale "SYMPERONIC PE/F68"® par la société "ICI" | 1 % |
| - Conservateur vendu sous la dénomination commerciale "KATHON CG"® par la société "ROHM & HAAS" | 0,1 % |
| - Eau | 92,9 % |

On a également préparé un placebo ayant la composition suivante :

| | |
|---|---|
| - Huile vendue sous la dénomination commerciale "MYGLYOL 812"® par la société "DYNAMIT NOBEL" | 5 % |
| - Cyanoacrylate de butyle | 1 % |
| - Tensio-actif vendu sous la dénomination commerciale "SYMPERONIC PE/F68"® par la société "ICI" | 1 % |
| - Conservateur vendu sous la dénomination commerciale "KATHON CG"® par la société "ROHM & HAAS" | 0,1 % |
| - Eau | 92,9 % |

On a évalué ensuite l'effet antiradicalaire de la dispersion aqueuse de nanoparticules et du placebo, in vivo, chez la souris sans poil en la soumettant à un rayonnement U.V. et en mesurant le taux de malonyldialdéhyde (M.D.A.) dans la peau, car la formation de M.D.A. est induite par le rayonnement U.V.

Les souris sont traitées par un nombre donné d'applications du produit testé. Chaque application est effectuée sur le dos de la souris avec 13,5 mg de préparation uniformément répartis sur 4,5 cm² et, lorsqu'il y a plusieurs applications, à 24 heures d'intervalle.

Une heure après la dernière application les souris sont soumises à un rayonnement U.V. à l'aide d'un appareil de type "BIOTRONIC U.V. 365/312 nm", qui émet un spectre avec des pics d'absorption à 365 nm (pour les U.V.A.) grâce à 2 tubes de 40 W et à 312 nm (pour les U.V.B.) grâce à un tube de 40 W. Les irradiations sont effectuées aux doses de 4,00 J/cm² en U.V.A. et 4,OO J/cm² en U.V.B. Trois heures après la fin de l'irradiation, les souris sont sacrifiées et la peau irradiée est découpée. Après plusieurs lavages, la surface d'application est essuyée et un fragment de 100 mg est introduit dans 1,8 ml de tampon phosphate, puis broyé à l'"ULTRA-TURRAX" pendant 1 mn. On dose ensuite le M.D.A., en milieu trichloroacétique et à chaud, par addition d'acide thiobarbiturique, qui se combine avec le M.D.A. pour former un complexe coloré rose, et par mesure de la concentration de ce complexe coloré au spectrophotomètre.

Les résultats sont donnés dans le tableau II:

**TABLEAU II**

| Test | Nombre d'applications avant irradiation | Taux de MDA (MOYENNE + SEM) en nanomoles MDA/mg protéines |
|---|---|---|
| Témoin sans irradiation | O | 2,655 ± O,O48 |
| Témoin irradié | O | 3,78O ± O,277 |
| Nanoparticules | 1 | 2,651 ± O,199 |
| " | 4 | 2,461 ± O,255 |
| Placebo | 1 | 4,19O ± O,323 |
| " | 4 | 3,8OO ± O,326 |

Sur ce tableau, on voit que l'effet de l'actif encapsulé dans les nanoparticules est maximum dès la première application, donc immédiat.

### EXEMPLE 3 :

Dans ce troisième exemple comparatif, on a préparé, selon le mode opératoire de l'exemple 4, une dispersion de nanoparticules selon l'invention, ayant la composition pondérale suivante :

| | |
|---|---|
| - Tensio-actif vendu sous la dénomination commerciale "PLURONIC F 68"® par la société "BASF" | 0,6 % |
| - Conservateur vendu sous la dénomination commerciale "KATHON CG"® par la société "ROHM & HAAS" | 0,05 % |
| - Butylcyanoacrylate | 0,3 % |
| - α-tocophérol radiomarqué | 1,5 % |
| - Eau q.s.p. | 100 % |

La concentration radioactive de la dispersion est de 12O »Ci/ml.

On a préparé, par ailleurs, une solution (non conforme à l'invention) contenant 1,5 % en poids d'α-tocophérol radiomarqué dans de l'huile de silicone vendue sous la dénomination commerciale "DC 200 FLUIDE"® par la société "DOW CORNING". La concentration radioactive de la solution est de 72 »Ci/ml.

On a évalué ensuite la pénétration de l'α-tocophérol de la dispersion de nanoparticules et de la solution dans l'huile de silicone dans les différentes couches de la peau. L'étude a été effectuée in vitro avec des cellules de Franz, cellules décrites dans "The Journal of Investigative Dermatology, volume 64 (1975), pages 190 - 195".

Des biopsies cutanées ont été préparées à partir de rats sans poils (hairless) femelles de 230 - 250 g. On a appliqué sur la surface de ces biopsies, coté stratum cornéum, des quantités identiques d'α-tocophérol marqué sous forme encapsulée dans des nanoparticules, d'une part, et sous forme de solution dans l'huile de silicone, d'autre part ; on les a laissées en contact pendant 24 heures. On a séparé ensuite mécaniquement le derme de l'épiderme et on a dosé l'α-tocophérol :

| | |
|---|---|
| - ayant traversé la peau | A |

| - retrouvé dans la peau | |
|---|---|
| dans l'épiderme et | B |
| dans le derme | C |
| - n'ayant pas pénétré dans la peau | D |

Les résultats obtenus sont rassemblés dans le tableau III ci-dessous où ils sont donnés en pour-cents en poids d'α-tocophérol pour 100 % d'α-tocophérol appliqué.

**TABLEAU III**

| Essais | α-tocophérol (% en poids) | | | |
|---|---|---|---|---|
| | A | B | C | D |
| Huile de silicone | 0,73 | 25,6 | 24,3 | 54,4 |
| Nanoparticules | 0,42 | 63,8 | 17,9 | 16,7 |

Sur ce tableau, on peut constater que la quantité d'α-tocophérol ayant traversé la peau est significativement plus élevée dans le cas de la solution dans l'huile de silicone que dans le cas de la dispersion de nanoparticules, mais reste très faible dans les deux cas. D'autre part, la quantité totale d'α-tocophérol retenue dans la peau (épiderme et derme) est beaucoup plus élevée dans le cas de la dispersion de nanoparticules que dans le cas de la solution dans l'huile de silicone, la majeure partie de l'α-tocophérol restant au niveau de l'épiderme dans le cas de la dispersion de nanoparticules.

### EXEMPLE 4 :

Dans un bécher de 125O ml, on dissout 1 g d'un mélange de condensats d'oxyde d'éthylène et d'oxyde de propylène (20 % - 80 % en poids), de poids moléculaires moyens 1750 et 8350 respectivement, vendu sous la dénomination commerciale "PLURONIC F-68"® par la société "BASF", dans 500 ml d'eau déminéralisée, sous l'agitation donnée par un barreau magnétique tournant à 400 tours par minute.

Dans cette phase aqueuse, on verse lentement, au goutte à goutte, 625 ml d'éthanol absolu, dans lequel on a préalablement dissous :
- 5 g d'α-tocophérol et
- 1 g de n-butyl cyanoacrylate.

On maintient l'agitation pendant deux heures, tout en régulant à 20°C la température du milieu réactionnel à l'aide d'un bain thermostaté. Après ces deux heures, on transfère la dispersion de nanoparticules obtenue dans un ballon rond de 1 litre que l'on place sur un évaporateur rotatif et l'on évapore l'éthanol et une partie de l'eau.

On récupère ainsi 95 g d'une dispersion colloïdale de nanoparticules contenant 5 g d'α-tocophérol encapsulé, dont le diamètre et l'indice de polydispersité (IP), mesurés au "nanosizer" (granulomètre Coultronics) sont respectivement de 190 nm et de 1.

L'indice IP est évalué sur une échelle de 0 à 9, O correspondant à une dispersion très homogène en tailles, alors qu'un indice de 9 indique un rapport élevé entre la taille des plus grosses particules et celle des plus petites.

On ajoute enfin 5 g d'une solution aqueuse contenant 0,1 g d'un agent conservateur, vendu sous la dénomination commerciale "KATHON CG"® par la société "ROHM & HAAS".

### EXEMPLE 5 :

On a préparé, selon le même mode opératoire que dans l'exemple 4, une dispersion aqueuse ayant la composition suivante :

| | |
|---|---|
| - Condensats d'oxyde d'éthylène et d'oxyde de propylène vendus sous la dénomination commerciale "PLURONIC F-68"® par la société "BASF" | 0,4 g |
| - Huile de carthame | 1,0 g |
| - Isohexyl cyanoacrylate | 0,4 g |
| - Eau q.s.p. | 100 g |

On obtient des nanoparticules, dont le diamètre moyen est de 25O nm, avec un indice IP égal à 2.

### EXEMPLE 6 :

On a préparé, selon le même mode opératoire que dans l'exemple 4, une dispersion aqueuse ayant la composition suivante :

| | |
|---|---|
| - Condensats d'oxyde d'éthylène et d'oxyde de propylène vendus sous la dénomination commerciale "PLURONIC F-68"® par la société "BASF" | 0,2 g |
| - Tétraéthyl-2-hexanoate de pentaérythritol | 1,0 g |
| - Butyl cyanoacrylate | 0,2 g |
| - Eau q.s.p. | 100 g |

On obtient des nanoparticules, dont le diamètre moyen est de 250 nm, avec un indice IP égal à 1.

### EXEMPLE 7 :

On a préparé, selon le même mode opératoire que dans l'exemple 4, une dispersion aqueuse ayant la composition suivante :

| | |
|---|---|
| - Condensats d'oxyde d'éthylène et d'oxyde de propylène vendus sous la dénomination commerciale "PLURONIC F-68"® par la société "BASF" | 0,4 g |
| - Triglycérides vendus sous la dénomination commerciale "MIGLYOL 812"® par la société "DYNAMIT NOBEL" | 1,0 g |
| - Palmitate de vitamine A | 0,02 g |
| - Butyl cyanoacrylate | 0,2 g |
| - Eau q.s.p | 100 g |

On obtient des nanoparticules, dont le diamètre moyen est de 240 nm, avec un indice IP égal à 2.

### EXEMPLE 8 :

Dans un bécher de 200 ml, on fait gonfler 0,3 g d'acide polyacrylique réticulé vendu sous la dénomination commerciale "CARBOPOL 940"® par la société "GOODRICH" dans 41,1 g d'eau contenant 0,1 g de parahydroxybenzoate de méthyle préalablement dissous. Cette opération est réalisée à la température de 80°C ; puis on revient progressivement à la température de 40°C. On ajoute 5 g d'eau contenant 0,1 g de gomme naturelle vendue sous la dénomination commerciale "AUBY GUM X2"® par la société "SATIA".

Après homogénéisation, on revient à la température ambiante et on ajoute 10 g de solution aqueuse obtenue par broyage de tissus placentaires animaux vendue sous la dénomination commerciale "PHYLDERM"® par la société "GATTEFOSSE" et
- 2 g de glycérol,
- 1 g d'hexylène glycol,
- 15 g d'eau de rose.

Après homogénéisation, on ajoute O,1 g du conservateur vendu sous la dénomination commerciale "DOWICIL 200"® par la société "DOW CHEMICAL", préalablement dissous dans 5 g d'eau. On ajoute 10 g de la dispersion aqueuse de nanoparticules préparée à l'exemple 4 et 10 g de la dispersion aqueuse de nanoparticules préparée selon l'exemple 7. Après homogénéisation, on ajoute 0,3 g de triéthanolamine. On obtient ainsi un sérum blanc bleuté, d'une viscosité égale à 1,6 poise et d'un pH égal à 7.

Ce sérum est appliqué une fois par jour sur la peau nettoyée du visage. On constate, après quinze jours d'application, un effet de lissage de la peau du visage.

### EXEMPLE 9 :

Dans un bécher de 200 ml, on dissout dans 25g d'eau de rose :
- 0,25 mg de colorant "FD et C BLUE 1" commercialisé par la société "ALLIED CHEMICAL" ; et
- 0,1 g de colorant "D et C YELLOW 10" commercialisé par la société "ANSTEAD".

On ajoute alors 30,2 g d'eau contenant 0,9 g de chlorure de sodium, puis 1,0 g de butylène glycol et 2,0 g de glycérol.

On homogénéise, puis on ajoute 20 g d'eau dans laquelle on a fait gonfler 0,05 g d'acide polyacrylique réticulé vendu sous la dénomination commerciale "CARBOPOL 1342"® par la société "GOODRICH".

Après homogénéisation, on ajoute alors 20 g de la dispersion aqueuse de nanoparticules obtenue à l'exemple 6. On ajoute enfin 0,05 g de triéthanolamine dissous dans 0,83 g d'eau. On obtient ainsi une lotion hydratante très fluide, de couleur verte et de pH égal à 6,7.

Cette lotion est appliquée deux fois par jour, après nettoyage du visage, en particulier chez des personnes à peau sèche. Après un mois d'application, on constate que la peau du visage est plus souple et que la sensation de tiraillement provoquée par la sécheresse de la peau est estompée.

### EXEMPLE 10 :

Dans la cuve en verre de 1 litre d'un homogénéiseur "COS 1000" commercialisé par la société "COSMETOCHEM", on verse 380,50 g d'eau florale dans laquelle on dissout :
- 0,5 g d'essence d'hamamelis sur support glycolique, commercialisée par la société "SOSHIBO",
- 2,5 g d'hexylène glycol,
- 10,0 g de glycérol, et
- 1,0 g de condensats d'oxyde d'éthylène et de propylène vendus sous la dénomination commerciale "PLURONIC F-68"® par la société "BASF".

On homogénéise cette solution au moyen d'un racleur tournant à la vitesse de 30 tours par minute et d'une turbine tournant à 90 tours par minute, la cuve de fabrication étant thermostatée à la température de 20°C. Pendant cette homogénéisation, on ajoute de façon continue, en 20 minutes, une solution alcoolique composée de :

| | |
|---|---|
| - Ethanol | 75 g |
| - Butyl cyanoacrylate | 0,5 g |
| - Acétate d'α-tocophérol | 5,0 g |

Après introduction complète de la phase alcoolique, on ramène la vitesse de la turbine à 70 tours par minute et on maintient cette agitation pendant deux heures.

On ajoute alors 24,25 g d'eau contenant à l'état dissous les agents conservateurs suivants :

| | |
|---|---|
| - Conservateur vendu sous la dénomination commerciale "DOWICIL 200"® par la société "DOW CHEMICAL" | 0,5 g |
| - Conservateur vendu sous la dénomination commerciale "KATHON CG"® par la société "ROHM & HAAS" | 0,25 g |

On obtient alors 500 g d'une lotion alcoolique. On mesure, à l'aide d'un granulomètre "BI 90" (vendu par la société "BROOKHAVEN"), le diamètre moyen des nanoparticules obtenues : il est de 225 nm ; l'indice de polydispersité est de 2.

Cette lotion est appliquée sur le corps tous les jours après la douche ou le bain, en particulier pendant les périodes d'expositions fréquentes au soleil. Elle exerce une action protectrice de la peau vis-à-vis des effets nocifs du rayonnement solaire.

### EXEMPLE 11 :

Dans un bécher en verre de 200 ml, on dissout dans 24 g d'huile de vaseline chauffée à la température de 65°C :
- 5 g d'alcool cétylique, et
- 3 g d'un monostéarate de polyéthylène-glycol à 50 moles d'oxyde d'éthylène vendu sous la dénomination commerciale "MYRJ 53"® par la société "ICI".

Après être revenu à la température de 50°C, on ajoute sous l'agitation donnée par un homogénéiseur "MORITZ", une phase aqueuse à même température constituée de :
- une solution, dans 44,7 g d'eau, de 0,3 g du mélange de conservateurs vendu sous la dénomination commerciale "ELESTAB 4112"® par la société "LSN", et
- 20 g de la dispersion aqueuse de nanoparticules obtenue dans l'exemple 4.

L'homogénéisation est maintenue pendant le refroidissement du produit à la température ambiante. On obtient ainsi 100 g d'une crème blanche et épaisse, qui a un effet protecteur vis-à-vis du rayonnement solaire.

Cette crème est appliquée quotidiennement, sur une peau parfaitement nettoyée ; elle est destinée plus particulièrement aux peaux exposées quotidiennement au rayonnement solaire.

### EXEMPLE 12 :

Dans un bécher en verre de 200 ml, on mélange, à la température de 85°C et à l'aide d'un homogénéiseur "MORITZ", les composés suivants :

| | |
|---|---|
| - Produit vendu sous la dénomination commerciale "MEXANYL GO"® par la société "CHIMEX" | 5,70 g |
| - Lanoline hydrogénée vendue sous la dénomination commerciale "SUPERSAT"® par la société "RITA" | 6,65 g |
| - Produit vendu sous la dénomination commerciale "MEXANYL GP"® fabriqué par la société "CHIMEX" | 2,00 g |

Après homogénéisation, ce premier mélange est dissous dans un second mélange constitué de :

| | |
|---|---|
| - Huile de purcellin liquide | 3,00 g |
| - Produit vendu sous la dénomination commerciale "Liquide base CB 1145"® par la société "CRODA" | 3,00 g |
| - Palmitate d'isopropyle | 4,75 g |
| - Huile de vaseline | 7,9 g |
| - Vaseline blanche | 15 g |
| - Perhydrosqualène | 0,4 g |

Ces deux mélanges sont homogénéisés à la température de 80°C puis refroidis à la température de 40°C. On ajoute alors 0,8 g de parfum vendu sous la dénomination commerciale "CHEMODERM 1008"® par la société "FIRMENICH".

Sous agitation et à la température de 40°C, on introduit ensuite une phase aqueuse constituée par le mélange d'une solution de 0,25 g du mélange d'agents conservateurs vendu sous la dénomination commerciale "ELESTAB 4110"® par la société "LSN" dans 29,9g d'eau et de 20 g de la dispersion aqueuse de nanoparticules obtenue à l'exemple 5.

On maintient cette agitation pendant 20 minutes et on ajoute alors 0,3 g de conservateur vendu sous la dénomination commerciale "GERMAL 115"® par la société "ROHM & HAAS", additionné de 0,35 g d'eau.

Après retour à la température ambiante, on passe le produit obtenu dans une broyeuse de type tricylindre.

On obtient ainsi une crème blanche, lisse, épaisse, destinée au soin des peaux très sèches et vieillies. Après une application biquotidienne de cette crème pendant 15 jours, on observe une amélioration de l'état de surface de la peau traitée.

### EXEMPLE 13

On a préparé, selon le même mode opératoire que dans l'exemple 4,une dispersion aqueuse ayant la composition suivante :

| | |
|---|---|
| - Condensat d'oxyde d'éthylène et d'oxyde de propylène vendu sous la dénomination commerciale "Pluronic F 68"® par la société "BASF" | 0,25 g |
| - Bisabolol | 0,5 g |
| - Acétate d'α-tocopherol | 0,5 g |
| - Butylcyanoacrylate | 0,2 g |
| - Eau qsp | 100 g |

On obtient des nanoparticules, dont le diamètre moyen est de 215nm avec un indice IP égal à 1.

### EXEMPLE 14

On a préparé selon le même mode opératoire que dans l'exemple 4, une dispersion aqueuse ayant la composition suivante :

| | |
|---|---|
| - Condensat d'oxyde d'éthylène et d'oxyde de propylène vendu sous la dénomination commerciale "Pluronic F 68"® par la société "BASF" | 0,25 g |
| - Mélange de decyl oleate, de farnesol, de linoleate d'éthyle et d'acétate de farnesyle vendue sous la dénomination commerciale "Unibiovit B 33"® par la société "Induchem" | 1,0 g |
| - Butylcyanoacrylate | 0,2 g |
| - Eau qsp | 100 g |

On obtient des nanocapsules dont le diamètre moyen est de 240nm avec un indice IP égal à 2.

### EXEMPLE 15

### 1) Préparation de la dispersion de vésicules :

Dans un bécher en verre de 100ml, on pèse 0,05g de dimyristyl phosphate et 0,95g de tensioactif non ionique de formule (I) : 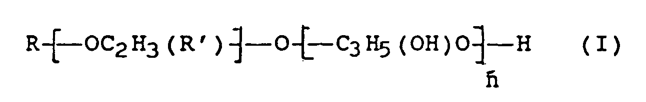
dans laquelle :
· -OC₂H₃(R')- désigne les structures suivantes, prises en mélange ou séparément : 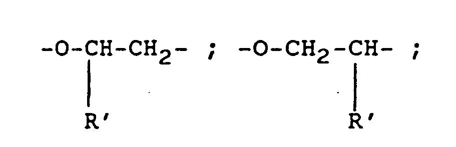
· -C₃H₅(OH)O- désigne les structures suivantes, prises en mélange ou séparément : -CH₂-CH(OH)-CH₂-O- ; 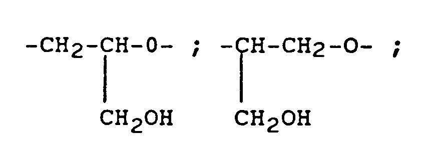
· n est une valeur statistique moyenne égale à 6;
· R = C₁₂H₂₅ et R' représente un mélange équimolaire des radicaux tétradécyle et hexadécyle.

On homogénéise ces deux lipides par chauffage sur une plaque chauffante à la température de 100°C, puis on refroidit le mélange à 40°C. On ajoute alors 27,6g d'eau dans laquelle on a préalablement dissous 3g de glycérine et 0,02g d'acide citrique. On homogénéise le tout par action d'un ultradisperseur de type "Virtis" pendant 2 minutes à la vitesse de 40.000t/mn, à la température de 40°C. On ajoute alors 3g d'eau dans laquelle on a préalablement dissous 0,1g de conservateur vendu sous la dénomination commerciale "Kathon CG"® par la société "Rohm et Haas", et 0,2g de conservateur vendu sous la dénomination commerciale "Germal 115"® par la société "Rohm et Haas".

On ajoute ensuite la phase grasse composée du mélange des produits suivants :

| | |
|---|---|
| - huile de macadamia | 9 g |
| - huile de silicone volatile | 7 g |
| - paramethoxy cinnamate d'éthyle hexyle vendu sous la dénomination commerciale "Parsol MCX"® par la société "Givaudan" | 0,5 g |
| - 2-hydroxy 4-méthoxybenzophenone vendu sous la dénomination commerciale "Uvinul M40"® par la société "BASF" | 0,5 g |
| - parahydroxybenzoate de propyle | 0,05 g |

On soumet le tout à l'action de l'ultradisperseur "Virtis" pendant 5 minutes à la température ambiante.

### 2) Mélange des dispersions de vésicules et de nanoparticules.

On ajoute alors 20g de la dispersion aqueuse de nanoparticules obtenue dans l'exemple 4. On ajoute ensuite 35g d'eau dans laquelle on a fait gonfler 0,65g d'acide carboxyvinylique vendu sous la dénomination commerciale "Carbopol 940"® par la société "Goodrich". Après homogénéisation, on ajoute enfin 0,65g de triéthanolamine diluée par 1,73g d'eau. On obtient ainsi une crème blanche, épaisse, d'aspect brillant destinée au soin du visage. Après une application de cette crème, une fois par jour pendant quinze jours, on observe une amélioration de l'état de surface de la peau traitée.

### EXEMPLE 16

Dans un bécher de 100ml, on dissout 250mg de condensat d'oxyde d'éthylène et d'oxyde de propylène (20-80%) de poids moléculaire moyen 1750 et 8350 respectivement, vendu sous la dénomination commerciale de "Pluronic F68"® par la Société "BASF", dans 50ml d'eau déminéralisée, sous l'agitation donnée par un barreau magnétique tournant à 400t/mn. Dans cette phase aqueuse thermostatée à la température de 40°C, on verse lentement 25ml d'acétone, dans lesquels on a préalablement dissous à la température de 40°C :
- 125 mg d'acide D,L polylactique vendu par la société "Polysciences"
- 500 mg d'acétate d'α-tocophérol vendu par la société "Roche"
- 2 mg de colorant "Nile Red" (Nile Blue A Oxazone) vendu par la société "Sigma".

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale fluide de nanoparticules, dont le diamètre moyen est de 295nm avec un indice IP égal à 2.

A l'examen microscopique en lumière fluorescente, on observe une population dense de nanoparticules fluorescentes à coeur, bien individualisées, homogènes en taille, agitées d'un mouvement brownien. Après 2 mois de conservation aux températures +4,25, 37 et 45°C, on n'observe pas de modification de l'aspect des nanoparticules à l'examen en lumière fluorescente, ni de variations importantes de leur taille (dans tous les cas, les variations sont inférieures à 10% du diamètre moyen initial).

### EXEMPLE 17

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation donnée par un barreau magnétique tournant à 400t/mn. Dans cette phase aqueuse thermostatée à la température de 40°C, on verse lentement 25ml d'acétone, dans lesquels on a préalablement dissous, à la température de 55°C, 125mg de lécithine de soja vendue sous la dénomination commerciale "Epikuron 170"® par la société "Lucas Meyer"; puis, après être revenu à la température de 40°C, on dissout :
- 125 mg d'acide D,L polylactique vendu par la société "Polysciences"
- 500 mg d'acétate d'α-tocophérol vendu par la société "Roche"
- 2 mg de colorant "Nile Red" (Nile Blue A Oxazone) vendu par la société "Sigma".

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale blanche, fluide de nanoparticules, dont le diamètre moyen est de 225nm avec un indice IP égal à 2.

A l'examen microscopique en lumière fluorescente, on observe une population dense de nanoparticules fluorescentes à coeur, bien individualisées, homogènes en taille, agitées d'un mouvement brownien. Après 2 mois de conservation aux températures de +4,25, 37 et 45°C, on n'observe pas de modification de l'aspect des nanoparticules à l'examen en microscopie de fluorescence, ni de variations importantes de leur taille (dans tous les cas, les variations sont inférieures à 10% du diamètre moyen initial).

### EXEMPLE 18

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation donnée par un barreau magnétique tournant à 400t/mn. Dans cette phase aqueuse thermostatée à la température de 40°C, on verse lentement 25ml d'acétone, dans lesquels on a préalablement dissous à la température de 40°C :
- 125 mg d'acide D,L polylactique vendu par la société "Polysciences"
- 500 mg d'acétate d'α-tocophérol vendu par la société "Roche".

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale blanche, fluide de nanoparticules, dont le diamètre moyen est de 310nm avec un indice IP égal à 2.

A l'examen microscopique, on observe une population dense de nanoparticules, bien individualisées, homogènes en taille, agitées d'un mouvement brownien. Après 2 mois de conservation aux températures de +4,25, 37, 45°C, on n'observe pas de modification de l'aspect des nanocapsules, ni de variations importantes de leur taille (dans tous les cas, les variations sont inférieures à 10% du diamètre moyen initial).

### EXEMPLE 19

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation douée par un barreau magnétique tournant à 400t/mn. Dans cette phase aqueuse thermostatée à la température de 40°C, on verse lentement 25ml d'acétone, dans lesquels on a préalablement dissous, à la température de 55°C, 250mg de lécithine de soja vendue sous la dénomination commerciale "Epikuron 170"® par la société "Lucas Meyer"; puis après être revenu à la température de 40°C, on dissout :
- 125 mg d'acide D,L Polylactique vendu par la société "Polysciences"
- 500 mg de paraméthoxycinnamate d'éthylhexyle commercialisé sous la dénomination commerciale "Parsol MCX"® par la société "Givaudan".

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale blanche, fluide de nanoparticules, dont le diamètre moyen est de 240nm avec un indice IP égal à 2.

A l'examen microscopique en lumière blanche, on observe une population dense de nanoparticules, bien individualisées, homogènes en taille, agitées d'un mouvement brownien. Après 2 mois de conservation aux températures de +4,25, 37 et 45°C, on n'observe pas de modification d'aspect des nanoparticules à l'examen microscopique, ni de variations importantes de leur taille (dans tous les cas, les variations sont inférieures à 10% du diamètre moyen initial).

### EXEMPLE 20

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation donnée par un barreau magnétique tournant à 400t/mn. Dans cette phase aqueuse, on verse lentement 25ml d'acétone, dans lesquels on a préalablement dissous 500mg d'acétate d'α-tocophérol et 125mg de polymère de cyanoacrylate de butyle obtenu par autopolymérisation préalable dans l'eau de monomère butylcyanoacrylate, isolation par centrifugation, puis séchage par lyophilisation. On maintient l'agitation pendant 2 heures à température ambiante. On transfère alors la dispersion de nanoparticules obtenue dans un ballon rond de 250ml que l'on place sur un évaporateur rotatif et on évapore l'acétone. On obtient ainsi une dispersion colloïdale, fluide de nanoparticules, dont le diamètre moyen est de 220nm avec un indice IP égal à 1.

A l'examen microscopique en lumière fluorescente, on observe une population dense de nanoparticules fluorescentes à coeur, bien individualisées, homogènes en taille, agitées d'un mouvement brownien. Après 2 mois de conservation aux températures de +4,25, 37 et 45°C, on n'observe pas de modification de l'aspect des nanoparticules à l'examen en lumière fluorescente, ni de variations importantes de leur taille (dans tous les cas, les variations sont inférieures à 10% du diamètre moyen initial).

### EXEMPLE 21

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation donnée par un barreau magnétique tournant à 400t/mn. Dans cette phase aqueuse thermostatée à la température de 40°C, on verse lentement 25ml d'acétone, dans lesquels on a préalablement dissous, à la température de 55°C, 250mg de lécithine de soja vendue sous la dénomination commerciale "Epikuron 170"® par la société "Lucas Meyer"; puis après être revenu à la température de 40°C, on dissout :
- 500 mg d'acétate d'α-tocopherol commercialisé par la société "Roche"
- 125 mg de copoly(D,L-lactide/glycolide) (dans le rapport molaire 50/50) commercialisé par la société "Dupont de Nemours" sous la dénomination commerciale "Medisorb T.M."®.

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale, fluide de nanoparticules, dont le diamètre moyen est de 210nm avec un indice IP égal à 1.

A l'examen microscopique en lumière fluorescente, on observe une population dense de nanoparticules fluorescentes à coeur, bien individualisées, homogènes en taille, agitées d'un mouvement brownien. Après 2 mois de conservation aux températures +4,25, 37 et 45°C, on n'observe pas de modification de l'aspect des nanoparticules à l'examen en lumière fluorescente, ni de variations importantes de leur taille (dans tous les cas, les variations sont inférieures à 10% du diamètre moyen initial).

### EXEMPLE 22

Dans un bécher de 100ml, on dissout 250mg de condensat d'oxyde d'éthylène et d'oxyde de propylène (20-80%), de poids moléculaire moyen 1750 et 8350 respectivement, vendu sous la dénomination commerciale de "Pluronic F68"® par la société "BASF", dans 50ml d'eau déminéralisée, sous l'agitation donnée par un barreau magnétique tournant à 400t/mn. Dans cette phase aqueuse thermostatée à la température de 40°C, on verse lentement 25ml d'acétone, dans lesquels on a préalablement dissous, à la température de 55°C, 250mg de lécithine de soja vendue sous la dénomination commerciale "Epikuron 170"® par la société "Lucas Meyer"; puis après être revenu à la température de 40°C, on dissout :
- 500 mg d'acétate d'α-tocophérol commercialisé par la société "Roche"
- 125 mg de polycaprolactone commercialisé par la société "Aldrich".

On maintient l'agitation pendant 2 heures à la température de 40°C, puis on revient à la température ambiante. On transfère alors la dispersion de nanoparticules obtenue dans un ballon rond de 250ml que l'on place sur un évaporateur rotatif et on évapore l'acétone. On obtient ainsi une dispersion colloïdale, fluide de nanoparticules, dont le diamètre moyen est de 195nm avec un indice IP égal à 2.

A l'examen microscopique en lumière blanche, on observe une population dense de nanoparticules, bien individualisées, homogènes en taille, agitées d'un mouvement brownien. Après 2 mois de conservation aux température de +4,25, 37 et 45°C, on n'observe pas de modification de l'aspect des nanoparticules à l'examen en lumière fluorescente, ni de variations importantes de leur taille (dans tous les cas, les variations sont inférieures à 10% du diamètre moyen initial).

### EXEMPLE 23

Dans un récipient cannelé en verre de 200ml, on pèse :
- 2,5 g d'un gel hydroalcoolique constitué de lécithine de soja (20%), d'éthanol absolu, (16 degrés alcooliques) et d'eau, commercialisé sous la dénomination commerciale "Natipide II"® par la société "Natterman"
- 0,01 g de palmitate d'ascorbyle
- 5,0 g de glycérine
- 17,5 g d'eau.

On homogénéise le tout à la température ambiante à l'aide d'un ultradisperseur "Virtis" pendant 3 minutes à la vitesse de 30000t/mn. On obtient ainsi une dispersion de liposomes, dont la taille moyenne est de 200nm.

On ajoute alors 1g de polyéthylène-glycol 400 et 3,0g de propylène-glycol: on mélange le tout à la température ambiante pendant 1 minute à l'ultradisperseur "Virtis". On ajoute 10g de solution aqueuse obtenue par broyage de tissus placentaires animaux, vendue sous la dénomination commerciale "Phylderm"® par la société "Gattefossé": on mélange le tout à la température ambiante pendant 1 minute à l'ultradisperseur "Virtis". On ajoute 0,2g de paraoxybenzoate de méthyle préalablement dissous dans 30,19g d'eau: on mélange le tout à la température ambiante pendant 1 minute à l'ultradisperseur "Virtis". On ajoute 0,1g de hyaluronate de sodium préalablement gonflé dans 10g d'eau, commercialisé par la société "Biotechnology".

On ajoute ensuite 0,25g du mélange d'acides carboxyvinyliques vendu sous la dénomination commerciale "Carbopole 940"® par lu société "Goodrich", préalablement gonflé dans 10g d'eau. On mélange le tout à la température ambiante pendant 3 minutes à l'ultradisperseur "Virtis" à la vitesse de 20000t/mn. On ajoute 0,25g de triéthanolamine et on homogénéise pendant 30 secondes à l'ultradisperseur "Virtis". On ajoute enfin 10g de dispersion de nanoparticules préparées à l'exemple 3. On homogénéise le tout à l'aide d'un homogénéiseur à palette de marque "Heidolph" à la vitesse de 800t/mn pendant 30 minutes.

On obtient ainsi un sérum de couleur beige, qui sera appliqué une fois par jour sur la peau nettoyée du visage, avant et après les périodes d'exposition au soleil. On constate que, malgré la fréquence élevée des expositions au soleil et leurs durées, la peau ne présente aucune trace d'irritation.

## Revendications (Revendications pour l'(les) Etat(s) contractant(s) suivant(s): AT, BE, CH, DE, DK, FR, GB, IT, LI, NL, SE)

1. Utilisation de nanoparticules polymères biodégradables encapsulant au moins un actif sous forme d'huile et/ou au moins un actif contenu dans une huile-support non active ou une huile active, l'actif étant choisi parmi ceux ayant une action pharmaceutique contre une affection se manifestant dans les couches supérieures de l'épiderme, pour l'obtention d'un médicament pour le traitement des couches supérieures de l'épiderme par application topique sur la peau.

2. Utilisation selon la revendication 1, caractérisée par le fait que les nanoparticules ont des dimensions comprises entre 10 et 1 000 nm.

3. Utilisation selon l'une des revendications 1 ou 2, caractérisée par le fait que le poids des nanoparticules chargées d'au moins un actif constitue de 0,1 % à 20 % du poids total de la composition.

4. Utilisation selon l'une des revendications 1 à 3, caractérisée par le fait que le polymère constitutif des nanoparticules est choisi dans le groupe formé par les polymères de cyanoacrylate d'alkyle (C₂-C₁₂), les poly-L-lactides, les poly-DL-lactides, les polyglycolides, les polycaprolactones, les polymères de l'acide 3-hydroxy butyrique, les copoly(DL-lactides et glycolides) et les copoly(glycolides et caprolactones).

5. Utilisation selon la revendication 4, caractérisée par le fait que le polymère constitutif des nanoparticules est un cyanoacrylate d'alkyle (C₂-C₆).

6. Utilisation selon la revendication 5, caractérisée par le fait que le radical alkyle du cyanoacrylate est choisi dans le groupe formé par les radicaux éthyle, n-butyle, hexyle, isobutyle et isohexyle.

7. Utilisation selon l'une des revendications 1 à 6, caractérisée par le fait que l'actif sous forme d'huile ou l'huile active est choisi dans le groupe formé par l'α -tocophérol, l'acétate d'α -tocophérol, les triglycérides riches en acide linoléique et/ou linolénique, le tétraéthyl-2-hexanoate de pentaérythritol, le clofibrate, le linoléate de tocophérol, l'huile de poisson, l'huile de noisette, le bisabolol, le farnesol, l'acétate de farnesyl, le linoléate d'éthyle, et le paraméthoxycinnamate d'éthyle hexyle.

8. Utilisation selon l'une des revendications 1 à 7, caractérisée par le fait que l'huile-support non active est choisie dans le groupe formé par les triglycérides, simples ou modifiés par oxyéthylénation, les huiles de silicone volatiles et leurs mélanges.

9. Utilisation selon l'une des revendications 1 à 8, caractérisée par le fait que l'actif contenu dans une huile est choisi dans le groupe formé par les émollients, les humectants, les agents anciradicalaires, les anti-inflammatoires, les vitamines les dépigmentants, les antiacnéiques, les antiséborrhéïques, les kératolytiques, les amincissants, et les agents de coloration de la peau.

10. Utilisation selon l'une des revendications 1 à 9, caractérisée par le fait que le rapport en poids entre le polymère biodégradable des nanoparticules et la phase huileuse active est compris entre 0,05 et 0,5.

11. Utilisation selon l'une des revendications 1 à 10, caractérisée par le fait que les nanoparticules sont dans une composition sous forme de sérum, de lotion, de gel aqueux, hydroalcoolique ou huileux, ou d'émulsion eau dans l'huile ou huile dans l'eau ou encore de dispersions aqueuses de vésicules, dont les lipides constitutifs sont des lipides ioniques ou des lipides non-ioniques ou un mélange de lipides ioniques et non-ioniques, avec ou sans phase huileuse.

12. Utilisation selon l'une des revendications 1 à 11, caractérisée par le fait qu'au moins une partie des nanoparticules comporte, autour de la membrane polymérique de la nanoparticule, au moins un feuillet lipidique.

13. Procédé de traitement cosmétique des couches supérieures de l'épiderme par application topique sur la peau, caractérisé par le fait que l'on applique sur la peau une composition contenant, dans un véhicule approprié, des nanoparticules polymères biodégradables encapsulant au moins un actif sous forme d'huile et/ou au moins un actif contenu dans une huile-support non active ou une huile active, l'actif étant choisi parmi ceux ayant une action cosmétique sur les couches supérieures de l'épiderme.

14. Procédé selon la revendication 13, caractérisé par le fait qu'on applique sur le peau une composition contenant des nanoparticules qui ont des dimensions comprises entre 10 et 1 000 nm.

15. Procédé selon l'une des revendications 13 ou 14, caractérisé par le fait qu'on applique sur la peau une composition dans laquelle le poids des nanoparticules chargées d'au moins un actif constitue de 0,1 % à 20 % du poids total de la composition.

16. Procédé selon l'une des revendications 13 à 15, caractérisé par le fait qu'on applique sur la peau une composition contenant des nanoparticules, dont le polymère constitutif est choisi dans le groupe formé par les polymères de cyanoacrylate d'alkyle (C₂-C₁₂), les poly-L-lactides, les poly-DL-lactides, les polyglycolides, les polycaprolactones, les polymères de l'acide 3-hydroxy butyrique, les copoly(DL-lactides et glycolides) et les copoly(glycolides et caprolactones).

17. Procédé selon la revendication 16, caractérisé par le fait que le polymère constitutif des nanoparticules est un cyanoacrylate d'alkyle (C₂-C₆).

18. Procédé selon la revendication 17, caractérisé par le fait que le radical alkyle du cyanoacrylate est choisi dans le groupe formé par les radicaux éthyle, n-butyle, hexyle, isobutyle et isohexyle.

19. Procédé selon l'une des revendications 13 à 18, caractérisé par le fait qu'on applique sur la peau une composition contenant des nanoparticules encapsulant un actif sous forme d'huile ou une huile active, choisi(e) dans le groupe formé par l'α - tocophérol, l'acétate d'α -tocophérol, les triglycérides riches en acide linoléique et/ou linolénique, le tétraéthyl-2-hexanoate de pentaérythritol, le clofibrate, le linoléate de tocophérol, l'huile de poisson, l'huile de noisette, le bisabolol, le farnesol, l'acétate de farnesyl, le linoléate d'éthyle, et le paraméthoxycinnamate d'éthyle hexyle.

20. Procédé selon l'une des revendications 13 à 19, caractérisé par le fait qu'on applique sur la peau une composition contenant des nanoparticules encapsulant une huile-support non active choisie dans le groupe formé par les triglycérides, simples ou modifiés par oxyéthylénation, les huiles de silicone volatiles et leurs mélanges.

21. Procédé selon l'une des revendications 13 à 20, caractérisé par le fait qu'on applique sur la peau une composition contenant des nanocapsules encapsulant un actif contenu dans une huile et choisi dans le groupe formé par les émollients, les humectants, les agents antiradicalaires, les anti-inflammatoires, les vitamines, les dépigmentants, les antiacnéiques, les antiséborrhéïques, les kératolytiques, les amincissants, et les agents de coloration de la peau.

22. Procédé selon l'une des revendications 13 à 21, caractérisé par le fait qu'on applique sur la peau une composition dans laquelle le rapport en poids entre le polymère biodégradable des nanoparticules et la phase huileuse active est compris entre 0,05 et 0,5.

23. Procédé selon l'une des revendications 13 à 22, caractérisé par le fait qu'on applique sur la peau une composition sous forme de sérum, de lotion, de gel aqueux, hydroalcoolique ou huileux, ou d'émulsion eau dans l'huile ou huile dans l'eau ou encore de dispersions aqueuses de vésicules, dont les lipides constitutifs sont des lipides ioniques ou des lipides non-ioniques ou un mélange de lipides ioniques et non-ioniques, avec ou sans phase huileuse.

24. Procédé selon l'une des revendications 13 à 23, caractérisé par le fait qu'on applique sur la peau une composition contenant des nanoparticules, dont au moins une partie comporte, autour de la membrane polymérique de la nanoparticule, au moins un feuillet lipidique.

25. Procédé de préparation des nanocapsules utilisées selon l'une des revendications 1 à 24, caractérisé par le fait que l'on effectue une polymérisation interfaciale d'une micro-émulsion d'huile dans un milieu hydroalcoolique en injectant, dans une phase aqueuse pouvant contenir un agent tensio-actif, un mélange constitué par l'(les) huile(s) à encapsuler, au moins un cyanoacrylate d'alkyle (C₂-C₁₂) et au moins un solvant pouvant contenir un agent tensio-actif, puis en évaporant le solvant.

26. Procédé selon la revendication 25, dans lequel la phase aqueuse contient un agent tensio-actif, caractérisé par le fait que le rapport pondéral entre l'agent tensio-actif utilisé, d'une part, et les matériaux constitutifs des nanoparticules chargées d'actif(s), d'autre part, est compris entre 0,01 et 0,5.

27. Procédé selon l'une des revendications 25 ou 26, caractérisé par le fait que l'on injecte, dans une phase aqueuse pouvant contenir un agent tensio-actif, un mélange contenant la (ou les) huile(s) à encapsuler, au moins un polymère et au moins un solvant pouvant contenir un agent tensio-actif, puis on évapore ledit solvant.

28. Procédé selon l'une des revendications 25 à 27, caractérisé par le fait que l'on utilise un agent tensio-actif pris dans le groupe formé par les tensio-actifs non-ioniques, les tensio-actifs ioniques et leurs mélanges.

29. Procédé selon la revendication 28, caractérisé par le fait que l'on choisit le tensio-actif non-ionique dans le groupe formé par les condensats de glycérol, d'oxyde d'éthylène et d'oxyde de propylène.

30. Procédé selon la revendication 28, caractérisé par le fait que l'on choisit comme tensio-actif ionique une lécithine.

## Revendications (Revendications pour l'(les) Etat(s) contractant(s) suivant(s): ES, GR)

1. Procédé de traitement cosmétique des couches supérieures de l'épiderme par application topique sur la peau, caractérisé par le fait que l'on applique sur la peau une composition contenant, dans un véhicule approprié, des nanoparticules polymères biodégradables encapsulant au moins un actif sous forme d'huile et/ou au moins un actif contenu dans une huile-support non active ou une huile active, l'actif étant choisi parmi ceux ayant une action cosmétique sur les couches supérieures de l'épiderme.

2. Procédé selon la revendication 1, caractérisé par le fait qu'on applique sur le peau une composition contenant des nanoparticules qui ont des dimensions comprises entre 10 et 1 000 nm.

3. Procédé selon l'une des revendications 1 ou 2, caractérisé par le fait qu'on applique sur la peau une composition dans laquelle le poids des nanoparticules chargées d'au moins un actif constitue de 0,1 % à 20 % du poids total de la composition.

4. Procédé selon l'une des revendications 1 à 3, caractérisé par le fait qu'on applique sur la peau une composition contenant des nanoparticules dont le polymère constitutif est choisi dans le groupe formé par les polymères de cyanoacrylate d'alkyle (C₂-C₁₂), les poly-L-lactides, les poly-DL-lactides, les polyglycolides, les polycaprolactones, les polymères de l'acide 3-hydroxy butyrique, les copoly(DL-lactides et glycolides) et les copoly(glycolides et caprolactones).

5. Procédé selon la revendication 4, caractérisé par le fait que le polymère constitutif des nanoparticules est un cyanoacrylate d'alkyle (C₂-C₆).

6. Procédé selon la revendication 5, caractérisé par le fait que le radical alkyle du cyanoacrylate est choisi dans le groupe formé par les radicaux éthyle, n-butyle, hexyle, isobutyle et isohexyle.

7. Procédé selon l'une des revendications 1 à 6, caractérisé par le fait qu'on applique sur la peau une composition contenant des nanoparticules encapsulant un actif sous forme d'huile ou une huile active, choisi(e) dans le groupe formé par l'α -tocophérol, l'acétate d'α -tocophérol, les triglycérides riches en acide linoléique et/ou linolénique, le tétraéthyl-2-hexanoate de pentaérythritol, le clofibrate, le linoléate de tocophérol, l'huile de poisson, l'huile de noisette, le bisabolol, le farnesol, l'acétate de farnesyl, le linoléate d'éthyle, et le paraméthoxycinnamate d'éthyle hexyle.

8. Procédé selon l'une des revendications 1 à 7, caractérisé par le fait qu'on applique sur la peau une composition contenant des nanoparticules encapsulant une huile-support non active choisie dans le groupe formé par les triglycérides, simples ou modifiés par oxyéthylénation, les huiles de silicone volatiles et leurs mélanges.

9. Procédé selon l'une des revendications 1 à 8, caractérisé par le fait qu'on applique sur la peau une composition contenant des nanocapsules encapsulant un actif contenu dans une huile et choisi dans le groupe formé par les émollients, les humectants, les agents antiradicalaires, les anti-inflammatoires, les vitamines, les dépigmentants, les antiacnéiques, les antiséborrhéïques, les kératolytiques, les amincissants, et les agents de coloration de la peau.

10. Procédé selon l'une des revendications 1 à 9, caractérisé par le fait qu'on applique sur la peau une composition dans laquelle le rapport en poids entre le polymère biodégradable des nanoparticules et la phase huileuse active est compris entre 0,05 et 0,5.

11. Procédé selon l'une des revendications 1 à 10, caractérisé par le fait qu'on applique sur la peau une composition sous forme de sérum, de lotion, de gel aqueux, hydroalcoolique ou huileux, ou d'émulsion eau dans l'huile ou huile dans l'eau ou encore de dispersions aqueuses de vésicules, dont les lipides constitutifs sont des lipides ioniques ou des lipides non-ioniques ou un mélange de lipides ioniques et non-ioniques, avec ou sans phase huileuse.

12. Procédé selon l'une des revendications 1 à 11, caractérisé par le fait qu'on applique sur la peau une composition contenant des nanoparticules, au moins une partie de ces nanoparticules comportant, autour de la membrane polymérique de la nanoparticule, au moins un feuillet lipidique.

13. Procédé de préparation des nanocapsules utilisées selon l'une des revendications 1 à 12, caractérisé par le fait que l'on effectue une polymérisation interfaciale d'une micro-émulsion d'huile dans un milieu hydroalcoolique en injectant, dans une phase aqueuse pouvant contenir un agent tensio-actif, un mélange constitué par l'(les) huile(s) à encapsuler, au moins un cyanoacrylate d'alkyle (C₂-C₁₂) et au moins un solvant pouvant contenir un agent tensio-actif, puis en évaporant le solvant.

14. Procédé selon la revendication 13, dans lequel la phase aqueuse contient un agent tensio-actif, caractérisé par le fait que le rapport pondéral entre l'agent tensio-actif utilisé, d'une part, et les matériaux constitutifs des nanoparticules chargées d'actif(s), d'autre part, est compris entre 0,01 et 0,5.

15. Procédé selon l'une des revendications 13 ou 14, caractérisé par le fait que l'on injecte, dans une phase aqueuse pouvant contenir un agent tensio-actif, un mélange contenant la (ou les) huile(s) à encapsuler, au moins un polymère et au moins un solvant pouvant contenir un agent tensio-actif, puis on évapore ledit solvant.

16. Procédé selon l'une des revendications 13 à 15, caractérisé par le fait que l'on utilise un agent tensio-actif pris dans le groupe formé par les tensio-actifs non-ioniques, les tensio-actifs ioniques et leurs mélanges.

17. Procédé selon la revendication 16, caractérisé par le fait que l'on choisit le tensio-actif non-ionique dans le groupe formé par les condensats de glycérol, d'oxyde d'éthylène et d'oxyde de propylène.

18. Procédé selon la revendication 16, caractérisé par le fait que l'on choisit comme tensio-actif ionique une lécithine.

## Claims (Claims for the following Contracting State(s): AT, BE, CH, DE, DK, FR, GB, IT, LI, NL, SE)

1. Use of biodegradable polymer nanoparticles encapsulating at least one active ingredient in the form or an oil and/or at least one active ingredient contained in an inactive carrier oil or an active oil, the active ingredient being selected from those having a pharmaceutical action against a disorder manifesting itself in the upper layers of the epidermis, for obtaining a medicinal product for the treatment of the upper layers of the epidermis by topical application to the skin.

2. Use according to Claim 1, characterized in that the nanoparticles are between 10 and 1,000 nm in size.

3. Use according to one of Claims 1 and 2, characterized in that the weight of the nanoparticles loaded with at least one active ingredient constitutes from 0.1 % to 20 % of the total weight of the composition.

4. Use according to one of Claims 1 to 3, characterized in that the polymer constituting the nanoparticles is selected from the group composed of polymers of (C₂-C₁₂) alkyl cyanoacrylate, poly-L-lactides, poly-DL-lactides, polyglycolides, polycaprolactones, polymers of 3-hydroxybutyric acid, copoly(DL-lactides/glycolides) and copoly(glycolides/caprolactones).

5. Use according to Claim 4, characterized in that the polymer constituting the nanoparticles is a (C₂-C₆) alkyl cyanoacrylate.

6. Use according to Claim 5, characterized in that the alkyl radical of the cyanoacrylate is selected from the group composed of ethyl, n-butyl, hexyl, isobutyl and isohexyl radicals.

7. Use according to one of Claims 1 to 6, characterized in that the active ingredient in the form of an oil or the active oil is selected from the group composed of α-tocopherol, α-tocopherol acetate, triglycerides rich in linoleic and/or linolenic acid(s), pentaerythritol tetra(2-ethylhexanoate), clofibrate, tocopherol linoleate, fish oil, hazelnut oil, bisabolol, farnesol, farnesyl acetate, ethyl linoleate and ethylhexyl para-methoxycinnamate.

8. Use according to one of Claims 1 to 7, characterized in that the inactive carrier oil is selected from the group composed of triglycerides, simple or modified by oxyethylenation volatile silicone oils and mixtures thereof.

9. Use according to one of Claims 1 to 8, characterized in that the active ingredient contained in an oil is selected from the group composed of emollients, humectants, free radical-inhibiting agents, anti-inflammatories, vitamins, depigmenting agents, anti-acne agents, antiseborrhoeics, keratolytics, slimming agents and skin colouring agents.

10. Use according to one of Claims 1 to 9, characterized in that the weight ratio of the biodegradable polymer of the nanoparticles to the active oily phase is between 0.05 and 0.5.

11. Use according to one of Claims 1 to 10, characterized in that the nanoparticles are in a composition in the form of a physiological fluid, a lotion, an aqueous, aqueous-alcoholic or oily gel or a water-in-oil or oil-in-water emulsion, or alternatively of aqueous dispersions of vesicles in which the constituent lipids are ionic or nonionic lipids or a mixture of ionic and nonionic lipids, with or without an oily phase.

12. Use according to one of Claims 1 to 11, characterized in that at least a part of the nanoparticles contains at least one lipid lamella around the polymer membrane of the nanoparticle.

13. Process for cosmetic treatment of the upper layers of the epidermis by topical application to the skin, characterized in that a composition containing, in a suitable vehicle, biodegradable polymer nanoparticles encapsulating at least one active ingredient in the form of an oil and/or at least one active ingredient contained in an inactive carrier oil or an active oil is applied to the skin, the active ingredient being selected from those having a cosmetic action on the upper layers of the epidermis.

14. Process according to Claim 13, characterized in that a composition containing nanoparticles which are between 10 and 1,000 nm in size is applied to the skin.

15. Process according to one of Claims 13 and 14, characterized in that a composition in which the weight of the nanoparticles loaded with at least one active ingredient constitutes from 0.1 % to 20 % of the total weight of the composition is applied to the skin.

16. Process according to one of Claims 13 to 15, characterized in that a composition containing nanoparticles the constituent polymer of which is selected from the group composed of polymers of (C₂-C₁₂) alkyl cyanoacrylate, poly-L-lactides, poly-DL-lactides, polyglycolides, polycaprolactones, polymers of 3-hydroxybutyric acid, copoly(DL-lactides/glycolides) and copoly(glycolides/caprolactones) is applied to the skin.

17. Process according to Claim 16, characterized in that the polymer constituting the nanoparticles is a (C₂-C₆) alkyl cyanoacrylate.

18. Process according to Claim 17, characterized in that the alkyl radical of the cyanoacrylate is selected from the group composed of ethyl, n-butyl, hexyl, isobutyl and isohexyl radicals.

19. Process according to one of Claims 13 to 18, characterized in that a composition containing nanoparticles encapsulating an active ingredient in the form of an oil or an active oil selected from the group composed of α-tocophorol, α-tocopherol acetate, triglycerides rich in linoleic and/or linolenic acid(s), pentaerythritol tetra(2-ethylhexanoate), clofibrate, tocopherol linoleate, fish oil, hazelnut oil, bisabolol, farnesol, farnesyl acetate, ethyl linoleate and ethylhexyl para-methoxycinnamate is applied to the skin.

20. Process according to one of Claims 13 to 19, characterized in that a composition containing nanoparticles encapsulating an inactive carrier oil selected from the group composed of triglycerides, simple or modified by oxyethylenation, volatile silicone oils and mixtures thereof is applied to the skin.

21. Process according to one of Claims 13 to 20, characterized in that a composition containing nanocapsules encapsulating an active ingredient contained in an oil and selected from the group composed of emollients, humectants, free radical-inhibiting agents, anti-inflammatories, vitamins, depigmenting agents, anti-acne agents, antiseborrhoeics, keratolytics, slimming agents and skin colouring agents is applied to the skin.

22. Process according to one of Claims 13 to 21, characterized in that a composition in which the weight ratio of the biodegradable polymer of the nanoparticles to the active oily phase is between 0.05 and 0.5 is applied to the skin.

23. Process according to one of Claims 13 to 22, characterized in that a composition in the form of a physiological fluid, a lotion, an aqueous, aqueous-alcoholic or oily gel or a water-in-oil or oil-in-water emulsion, or alternatively of aqueous dispersions of vesicles in which the constituent lipids are ionic or nonionic lipids or a mixture of ionic and nonionic lipids, with or without an oily phase is applied to the skin.

24. Process according to one of Claims 13 to 23, characterized in that a composition containing nanoparticles, at least a part of which contains at least ono lipid lamella around the polymer membrane of the nanoparticle, is applied to the skin.

25. Process for preparing nanocapsules used according to one of Claims 1 to 24, characterized in that an interfacial polymerization of a microemulsion of oil in an aqueous-alcoholic medium is preferred by injecting, into an aqueous phase which can contain a surfactant, a mixture consisting of the oil(s) to be encapsulated, at least one (C₂-C₁₂) alkyl cyanoacrylate and at least one solvent which can contain a surfactant, and then evaporating off the solvent.

26. Process according to Claim 25, in which the aqueous phase contains a surfactant, characterized in that the weight ratio of the surfactant used on the one hand, to the materials constituting the nanoparticles loaded with active ingredient(s) on the other hand, is between 0.01 and 0.5.

27. Process according to one of Claims 25 and 26, characterized in that a mixture containing the oil(s) to be encapsulated, at least one polymer and at least one solvent which can contain a surfactant is injected into an aqueous phase which can contain a surfactant, and the said solvent is then evaporated off.

28. Process according to one of Claims 25 to 27, characterized in that a surfactant taken from the group composed of nonionic surfactants, ionic surfactants and mixtures thereof is used.

29. Process according to Claim 28, characterized in that the nonionic surfactant is selected from the group composed of the condensates of glycerol, ethylene oxide and propylene oxide.

30. Process according to Claim 28, characterized in that a lecithin is selected as an ionic surfactant.

## Claims (Claims for the following Contracting State(s): ES, GR)

1. Process for cosmetic treatment of the upper layers of the epidermis, by topical application to the skin, characterized in that a composition containing, in a suitable vehicle, biodegradable polymer nanoparticles encapsulating at least one active ingredient in the form of an oil and/or at least one active ingredient contained in an inactive carrier oil or an active oil is applied to the skin, the active ingredient being selected from those having a cosmetic action on the upper layers of the epidermis.

2. Process according to Claim 1, characterized in that a composition containing nanoparticles which are between 10 and 1,000 nm in size is applied to the skin.

3. Process according to one of Claims 1 and 2, characterized in that a composition in which the weight of the nanoparticles loaded with at least one active ingredient constitutes from 0.1 % to 20 % of the total weight of the composition is applied to the skin.

4. Process according to one of Claims 1 to 3, characterized in that a composition containing nanoparticles the constituent polymer of which is selected from the group composed of polymers of (C₂-C₁₂) alkyl cyanoacrylate, poly-L-lactides, poly-DL-lactides, polyglycolides, polycaprolactones, polymers of 3-hydroxybutyric acid, copoly(DL-lactides/glycolides) and copoly(glycolides/caprolactones) is applied to the skin.

5. Process according to Claim 4, characterized in tat the polymer constituting the nanoparticles is a (C₂-C₆) alkyl cyanoacrylate.

6. Process according to Claim 5, characterized in that the alkyl radical of the cyanoacrylate is selected from the group composed of ethyl, n-butyl, hexyl, isobutyl and isohexyl radicals.

7. Process according to one of Claims 1 to 6, characterized in that a composition containing nanoparticles encapsulating an active ingredient in the form of an oil or an active oil selected from the group composed of α-tocopherol, α-tocopherol acetate, triglycerides rich in linoleic and/or linolenic acid(s), pentaerythritol tetra(2-ethylhexanoate), clofibrate, tocopherol linoleate, fish oil, hazelnut oil, bisabolol, farnesol, farnesyl acetate, ethyl linoleate and ethylhexyl para-methoxycinnamate is applied to the skin.

8. Process according to one of Claims 1 to 7, characterized in that a composition containing nanoparticles encapsulating an inactive carrier oil selected from the group composed of triglycerides, simple or modified by oxyethylenation, volatile silicone oils and mixtures thereof is applied to the skin.

9. Process according to one of Claims 1 to 8, characterized in that a composition containing nanocapsules encapsulating an active ingredient contained in an oil and selected from the group composed of emollients, humectants, free radical-inhibiting agents, anti-inflammatories, vitamins, depigmenting agents, anti-acne agents, antiseborrhoeics, keratolytics, slimming agents and skin colouring agents is applied to the skin.

10. Process according to one of Claims 1 to 9, characterized in that a composition in which the weight ratio of the biodegradable polymer of the nanoparticles to the active oily phase is between 0.05 and 0.5 is applied to the skin.

11. Process according to one of Claims 1 to 10, characterized in that a composition in the form of a physiological fluid, a lotion, an aqueous, aqueous-alcoholic or oily gel or a water-in-oil or oil-in-water emulsion, or alternatively of aqueous dispersions of vesicles in which the constituent lipids are ionic or nonionic lipids or a mixture of ionic and nonionic lipids, with or without an oily phase is applied to the skin.

12. Process according to one of Claims 1 to 11, characterized in that a composition containing nanoparticles is applied to the skin, at least a part of these nanoparticles containing at least one lipid lamella around the polymer membrane of the nanoparticle.

13. Process for preparing nanocapsules used according to one of Claims 1 to 12, characterized in that an interfacial polymerization of a microemulsion of oil in an aqueous-alcoholic medium is performed by injecting, into an aqueous phase which can contain a surfactant, a mixture consisting of the oil(s) to be encapsulated, at least one (C₂-C₁₂) alkyl cyanoacrylate and at least one solvent which can contain a surfactant, and then evaporating off the solvent.

14. Process according to Claim 13, in which the aqueous phase contains a surfactant, characterized in that the weight ratio of the surfactant used on the one hand, to the materials constituting the nanoparticles loaded with active ingredient(s) on the other hand, is between 0.01 and 0.5.

15. Process according to one of Claims 13 and 14, characterized in that, a mixture containing the oil(s) to be encapsulated, at least one polymer and at least one solvent which can contain a surfactant is injected into an aqueous phase which can contain a surfactant, and the said solvent is then evaporated off.

16. Process according to one of Claims 13 to 15, characterized in that a surfactant taken from the group composed of nonionic surfactants, ionic surfactants and mixtures thereof is used.

17. Process according to Claim 16, characterized in that the nonionic surfactant is selected from the group composed of the condensates of glycerol, ethylene oxide and propylene oxide.

18. Process according to Claim 16, characterized in that a lecithin is selected as an ionic surfactant.

## Patentansprüche (Patentansprüche für folgende(n) Vertragsstaat(en): AT, BE, CH, DE, DK, FR, GB, IT, LI, NL, SE)

1. Verwendung von bioabbaubaren Polymernanopartikeln, die wenigstens einen Wirkstoff in Form eines Öls und/oder wenigstens einen in einem nicht-aktiven Trägeröl oder einem aktiven Öl enthaltenen Wirkstoff einkapseln, wobei der Wirkstoff unter solchen mit pharmazeutischer Wirkung gegen eine Erkrankung ausgewählt ist, die sich in den oberen Schichten der Epidermis manifestiert, zur Herstellung eines Medikamentes zur Behandlung der oberen Schichten der Epidermis durch topische Applikation auf die Haut.

2. Verwendung nach Anspruch 1, dadurch gekennzeichnet, daß die Nanopartikel eine Größe im Bereich von 10 bis 1000 nm besitzen.

3. Verwendung nach einem der Ansprüche 1 oder 2, dadurch gekennzeichnet, daß das Gewicht der mit wenigstens einem Wirkstoff beladenen Nanopartikel 0,1 bis 20 % des Gesamtgewichtes des Mittels ausmacht.

4. Verwendung nach einem der Ansprüche 1 bis 3, dadurch gekennzeichnet, daß das die Nanopartikel bildende Polymer ausgewählt ist unter (C₂-C₁₂)-Alkylcyanoacrylatpolymeren, Poly-L-lactiden, Poly-DL-lactiden, Polyglycoliden, Polycaprolactonen, 3-Hydroxybuttersäurepolymeren, Copoly(DL-lactiden und glycoliden) und Copoly(glycoliden und caprolactonen).

5. Verwendung nach Anspruch 4, dadurch gekennzeichnet, daß das die Nanopartikel bildende Polymer ein (C₂-C₆)-Alkylcyanoacrylat ist.

6. Verwendung nach Anspruch 5, dadurch gekennzeichnet, daß der Alkylrest des Cyanoacrylats ausgewählt ist unter einem Ethyl-, n-Butyl-, Hexyl-, Isobutyl- und Isohexylrest.

7. Verwendung nach einem der Ansprüche 1 bis 6, dadurch gekennzeichnet, daß der Wirkstoff in Form eines Öls oder das aktive Öl ausgewählt ist unter α-Tocopherol, α-Tocopherolacetat, Triglyceriden, die reich an Linolsäure und/oder Linolensäure sind, Pentaerythrit-tetraethyl-2-hexanoat, Clofibrat, Tocopherollinoleat, Fischöl, Nußöl, Bisabolol, Farnesol, Farnesylacetat, Ethyllinoleat und Ethylhexyl-p-methoxycinnamat.

8. Verwendung nach einem der Ansprüche 1 bis 7, dadurch gekennzeichnet, daß das nicht-aktive Trägeröl ausgewählt ist unter einfachen oder durch Oxyethylenierung modifizierten Triglyceriden, flüchtigen Siliconölen und Gemischen davon.

9. Verwendung nach einem der Ansprüche 1 bis 8, dadurch gekennzeichnet, daß der in einem Öl enthaltende Wirkstoff ausgewählt ist unter Emollienzien, Befeuchtungsmitteln, Mitteln gegen Radikale, antiinflammatorischen Mitteln, Vitaminen, Depigmentierungsmitteln, Antiaknemitteln, Antiseborrhöemitteln, keratolytischen Mitteln, dünnmachenden Mitteln und Mitteln zur Färbung der Haut.

10. Verwendung nach einem der Ansprüche 1 bis 9, dadurch gekennzeichnet, daß das Gewichtsverhältnis zwischen dem bioabbaubaren Polymer der Nanopartikel und der aktiven Ölphase im Bereich von 0,05 bis 0,5 liegt.

11. Verwendung nach einem der Ansprüche 1 bis 10, dadurch gekennzeichnet, daß die Nanopartikel in einer Zusammensetzung in Form eines Serums, einer Lotion, eines wäßrigen, wäßrigalkoholischen oder öligen Gels oder einer Wasser-in-Öl- oder Öl-in-Wasser-Emulsion oder auch einer wäßrigen Dispersion von Vesikeln, deren Lipidbestandteile ionische Lipide oder nicht-ionische Lipide oder ein Gemisch ionischer und nicht-ionischer Lipide sind, mit oder ohne Ölphase vorhanden sind.

12. Verwendung nach einem der Ansprüche 1 bis 11, dadurch gekennzeichnet, daß wenigstens ein Teil der Nanopartikel um die Polymermembran wenigstens ein Lipidplättchen aufweist.

13. Verfahren zur kosmetischen Behandlung der oberen Epidermisschichten durch topische Applikation auf die Haut, dadurch gekennzeichnet, daß man auf die Haut ein Mittel aufträgt, das in einem geeigneten Träger bioabbaubare Polymernanopartikel enthält, die wenigstens einen Wirkstoff in Form eines Öls und/oder wenigstens einen in einem nicht-aktiven Trägeröl oder einem aktiven Öl enthaltenen Wirkstoff einkapsein, wobei der Wirkstoff unter solchen mit kosmetischer Wirkung auf die oberen Schichten der Epidermis ausgewählt ist.

14. Verfahren nach Anspruch 13, dadurch gekennzeichnet, daß man auf die Haut ein Mittel aufträgt, das die Nanopartikel in einer Größe im Bereich von 10 bis 1000 nm enthält.

15. Verfahren nach einem der Ansprüche 13 oder 14, dadurch gekennzeichnet, daß man auf die Haut ein Mittel aufträgt, bei dem das Gewicht der mit wenigstens einem Wirkstoff beladenen Nanopartikel 0,1 bis 20 % des Gesamtgewichtes des Mittels ausmacht.

16. Verfahren nach einem der Ansprüche 13 bis 15, dadurch gekennzeichnet, daß man auf die Haut ein Mittel aufträgt, das Nanopartikel enthält, deren Polymerbestandteil ausgewählt ist unter (C₂-C₁₂)-Alkylcyanoacrylatpolymeren, Poly-L-lactiden, Poly-DL-lactiden, Polyglycoliden, Polycaprolactonen, 3-Hydroxybuttersäurepolymeren, Copoly(DL-lactiden und glycoliden) und Copoly(glycoliden und caprolactonen).

17. Verfahren nach Anspruch 16, dadurch gekennzeichnet, daß das die Nanopartikel bildende Polymer ein (C₂-C₆)-Alkylcyanoacrylat ist.

18. Verfahren nach Anspruch 17, dadurch gekennzeichnet, daß der Alkylrest des Cyanoacrylats ausgewählt ist unter einem Ethyl-, n-Butyl-, Hexyl-, Isobutyl- und Isohexylrest.

19. Verfahren nach einem der Ansprüche 13 bis 18, dadurch gekennzeichnet, daß man auf die Haut ein Mittel aufträgt, das Nanopartikel enthält, welche einen Wirkstoff in Form eines Öls oder ein aktives Öl einkapseln, das (die) ausgewählt ist(sind) unter α-Tocopherol, α-Tocopherolacetat, Triglyceriden, die reich an Linolsäure und/oder Linolensäure sind, Pentaerythrittetraethyl-2-hexanoat, Clofibrat, Tocopherollinoleat, Fischöl, Nußöl, Bisabolol, Farnesol, Farnesylacetat, Ethyllinoleat und Ethylhexyl-p-methoxycinnamat.

20. Verfahren nach einem der Ansprüche 13 bis 19, dadurch gekennzeichnet, daß man auf die Haut ein Mittel aufträgt, das Nanopartikel enthält, welche ein nicht-aktives Trägeröl einkapseln, das ausgewählt ist unter einfachen oder durch Oxyethylenierung modifizierten Triglyceriden, flüchtigen Siliconölen oder Gemischen davon.

21. Verfahren nach einem der Ansprüche 13 bis 20, dadurch gekennzeichnet, daß man auf die Haut ein Mittel aufträgt, das Nanokapseln enthält, welche einen Wirkstoff einkapseln, der in einem Öl enthalten und ausgewählt ist unter Emollienzien, Befeuchtungsmitteln, Mitteln gegen Radikale, antiinflammatorischen Mitteln, Vitaminen, Depigmentierungsmitteln, Antiaknemitteln, Antiseborrhöemitteln, keratolytischen Mitteln, dünnmachenden Mitteln und Mitteln zur Färbung der Haut.

22. Verfahren nach einem der Ansprüche 13 bis 21, dadurch gekennzeichnet, daß man auf die Haut ein Mittel aufträgt, bei dem das Gewichtsverhältnis zwischen dem bioabbaubaren Polymer der Nanopartikel und der aktiven Ölphase im Bereich von 0,05 bis 0,5 liegt.

23. Verfahren nach einem der Ansprüche 13 bis 22, dadurch gekennzeichnet, daß man auf die Haut ein Mittel in Form eines Serums, einer Lotion, eines wäßrigen, wäßrig-alkoholischen oder öligen Gels oder einer Wasser-in-Öl- oder Öl-in-Wasser-Emulsion oder einer wäßrigen Dispersion von Vesikeln mit oder ohne Ölphase, deren Lipidbestandteile ionische Lipide oder nicht-ionische Lipide oder ein Gemisch von ionischen und nicht-ionischen Lipiden sind, aufträgt.

24. Verfahren nach einem der Ansprüche 13 bis 23, dadurch gekennzeichnet, daß man auf die Haut ein Mittel aufträgt, das Nanopartikel enthält, von denen wenigstens ein Teil um die Polymermembran wenigstens ein Lipidplättchen aufweist.

25. Verfahren zur Herstellung der gemäß einem der Ansprüche 1 bis 24 verwendeten Nanokapseln, dadurch gekennzeichnet, daß man eine Grenzflächenpolymerisation einer Ölmikroemulsion in einem wäßrig-alkoholischen Milieu durchführt, indem man in eine wäßrige Phase, die ein grenzflächenaktives Mittel enthalten kann, ein Gemisch aus dem(den) einzukapselnden Öl(Ölen), wenigstens einem (C₂-C₁₂)-Alkylcyanoacrylat und wenigstens einem Lösungsmittel, das ein grenzflächenaktives Mittel enthalten kann, injiziert und anschließend das Lösungsmittel verdampft.

26. Verfahren nach Anspruch 25, bei dem die wäßrige Phase das grenzflächenaktive Mittel enthält, dadurch gekennzeichnet, daß das Gewichtsverhältnis zwischen dem zur Anwendung kommenden grenzflächenaktiven Mittel und den Materialbestandteilen der mit Wirkstoff(en) beladenen Nanopartikel im Bereich von 0,01 bis 0,5 liegt.

27. Verfahren nach einem der Ansprüche 25 oder 26, dadurch gekennzeichnet, daß man in eine wäßrige Phase, welche ein grenzflächenaktives Mittel enthalten kann, ein Gemisch, enthaltend das einzukapselnde Öl (oder die einzukapselnden Öle), wenigstens ein Polymer und wenigstens ein Lösungsmittel, das ein grenzflächenaktives Mittel enthalten kann, injiziert und anschließend das erwähnte Lösungsmittel verdampft.

28. Verfahren nach einem der Ansprüche 25 bis 27, dadurch gekennzeichnet, daß man ein grenzflächenaktives Mittel verwendet, das ausgewählt ist unter nicht-ionischen grenzflächenaktiven Mitteln, ionischen grenzflächenaktiven Mitteln und Gemischen davon.

29. Verfahren nach Anspruch 28, dadurch gekennzeichnet, daß das nicht-ionische grenzflächenaktive Mittel ausgewählt ist unter Kondensaten von Glycerin, Ethylenoxid und Propylenoxid.

30. Verfahren nach Anspruch 28, dadurch gekennzeichnet, daß man als ionisches grenzflächenaktives Mittel Lecithin auswählt.

## Patentansprüche (Patentansprüche für folgende(n) Vertragsstaat(en): ES, GR)

1. Verfahren zur kosmetischen Behandlung der oberen Epidermisschichten durch topische Applikation auf die Haut, dadurch gekennzeichnet, daß man auf die Haut ein Mittel aufträgt, das in einem geeigneten Träger bioabbaubare Polymernanopartikel enthält, die wenigstens einen Wirkstoff in Form eines Öls und/oder wenigstens einen in einem nicht-aktiven Trägeröl oder einem aktiven Öl enthaltenen Wirkstoff einkapseln, wobei der Wirkstoff unter solchen mit kosmetischer Wirkung auf die oberen Schichten der Epidermis ausgewählt ist.

2. Verfahren nach Anspruch 1, dadurch gekennzeichnet, daß man auf die Haut ein Mittel aufträgt, das die Nanopartikel in einer Größe im Bereich von 10 bis 1000 nm enthält.

3. Verfahren nach einem der Ansprüche 1 oder 2, dadurch gekennzeichnet, daß man auf die Haut ein Mittel aufträgt, bei dem das Gewicht der mit wenigstens einem Wirkstoff beladenen Nanopartikel 0,1 bis 20 % des Gesamtgewichtes des Mittels ausmacht.

4. Verfahren nach einem der Ansprüche 1 bis 3, dadurch gekennzeichnet, daß man auf die Haut ein Mittel aufträgt, das Nanopartikel enthält, deren Polymerbestandteil ausgewählt ist unter (C₂-C₁₂)-Alkylcyanoacrylatpolymeren, Poly-L-lactiden, Poly-DL-lactiden, Polyglycoliden, Polycaprolactonen, 3-Hydroxybuttersäurepolymeren, Copoly(DL-lactiden und glycoliden) und Copoly(glycoliden und caprolactonen).

5. Verfahren nach Anspruch 4, dadurch gekennzeichnet, daß das die Nanopartikel bildende Polymer ein (C₂-C₆)-Alkylcyano-acrylat ist.

6. Verfahren nach Anspruch 5, dadurch gekennzeichnet, daß der Alkylrest des Cyanoacrylats ausgewählt ist unter einem Ethyl-, n-Butyl-, Hexyl-, Isobutyl- und Isohexylrest.

7. Verfahren nach einem der Ansprüche 1 bis 6, dadurch gekennzeichnet, daß man auf die Haut ein Mittel aufträgt, das Nanopartikel enthält, welche einen Wirkstoff in Form eines Öls oder ein aktives Öl einkapseln, das (die) ausgewählt ist(sind) unter α-Tocopherol, α-Tocopherolacetat, Triglyceriden, die reich an Linolsäure und/oder Linolensäure sind, Pentaerythrittetraethyl-2-hexanoat, Clofibrat, Tocopherollinoleat, Fischöl, Nußöl, Bisabolol, Farnesol, Farnesylacetat, Ethyllinoleat und Ethylhexyl-p-methoxycinnamat.

8. Verfahren nach einem der Ansprüche 1 bis 7, dadurch gekennzeichnet, daß man auf die Haut ein Mittel aufträgt, das Nanopartikel enthält, welche ein nicht-aktives Trägeröl einkapseln, das ausgewählt ist unter einfachen oder durch Oxyethylenierung modifizierten Triglyceriden, flüchtigen Siliconölen oder Gemischen davon.

9. Verfahren nach einem der Ansprüche 1 bis 8, dadurch gekennzeichnet, daß man auf die Haut ein Mittel aufträgt, das Nanokapseln enthält, welche einen Wirkstoff einkapseln, der in einem Öl enthalten und ausgewählt ist unter Emollienzien, Befeuchtungsmitteln, Mitteln gegen Radikale, antiinflammatorischen Mitteln, Vitaminen, Depigmentierungsmitteln, Antiaknemitteln, Antiseborrhöemitteln, keratolytischen Mitteln, dünnmachenden Mitteln und Mitteln zur Färbung der Haut.

10. Verfahren nach einem der Ansprüche 1 bis 9, dadurch gekennzeichnet, daß man auf die Haut ein Mittel aufträgt, bei dem das Gewichtsverhältnis zwischen dem bioabbaubaren Polymer der Nanopartikel und der aktiven Ölphase im Bereich von 0,05 bis 0,5 liegt.

11. Verfahren nach einem der Ansprüche 1 bis 10, dadurch gekennzeichnet, daß man auf die Haut ein Mittel in Form eines Serums, einer Lotion, eines wäßrigen, wäßrig-alkoholischen oder öligen Gels oder einer Wasser-in-Öl- oder Öl-in-Wasser-Emulsion oder einer wäßrigen Dispersion von Vesikeln mit oder ohne Ölphase, deren Lipidbestandteile ionische Lipide oder nicht-ionische Lipide oder ein Gemisch von ionischen und nicht-ionischen Lipiden sind, aufträgt.

12. Verfahren nach einem der Ansprüche 1 bis 11, dadurch gekennzeichnet, daß man auf die Haut ein Mittel aufträgt, das Nanopartikel enthält, von denen wenigstens ein Teil um die Polymermembran wenigstens ein Lipidplättchen aufweist.

13. Verfahren zur Herstellung der gemäß einem der Ansprüche 1 bis 12 verwendeten Nanokapseln, dadurch gekennzeichnet, daß man eine Grenzflächenpolymerisation einer Ölmikroemulsion in einem wäßrig-alkoholischen Milieu durchführt, indem man in eine wäßrige Phase, die ein grenzflächenaktives Mittel enthalten kann, ein Gemisch aus dem(den) einzukapselnden Öl(Ölen), wenigstens einem (C₂-C₁₂)-Alkylcyanoacrylat und wenigstens einem Lösungsmittel, das ein grenzflächenaktives Mittel enthalten kann, injiziert und anschließend das Lösungsmittel verdampft.

14. Verfahren nach Anspruch 13, bei dem die wäßrige Phase das grenzflächenaktive Mittel enthält, dadurch gekennzeichnet, daß das Gewichtsverhältnis zwischen dem zur Anwendung kommenden grenzflächenaktiven Mittel und den Materialbestandteilen der mit Wirkstoff(en) beladenen Nanopartikel im Bereich von 0,01 bis 0,5 liegt.

15. Verfahren nach einem der Ansprüche 13 oder 14, dadurch gekennzeichnet, daß man in eine wäßrige Phase, welche ein grenzflächenaktives Mittel enthalten kann, ein Gemisch, enthaltend das einzukapselnde Öl (oder die einzukapselnden Öle), wenigstens ein Polymer und wenigstens ein Lösungsmittel, das ein grenzflächenaktives Mittel enthalten kann, injiziert und anschließend das erwähnte Lösungsmittel verdampft.

16. Verfahren nach einem der Ansprüche 13 bis 15, dadurch gekennzeichnet, daß man ein grenzflächenaktives Mittel verwendet, das ausgewählt ist unter nicht-ionischen grenzflächenaktiven Mitteln, ionischen grenzflächenaktiven Mitteln und Gemischen davon.

17. Verfahren nach Anspruch 16, dadurch gekennzeichnet, daß das nicht-ionische grenzflächenaktive Mittel ausgewählt ist unter Kondensaten von Glycerin, Ethylenoxid und Propylenoxid.

18. Verfahren nach Anspruch 16, dadurch gekennzeichnet, daß man als ionisches grenzflächenaktives Mittel Lecithin auswählt.
